# EUROPÄISCHE PATENTANMELDUNG

(11) **EP 0 832 638 A2**
(43) Veröffentlichungstag der Anmeldung: **01.04.1998**
(21) Anmeldenummer: 97115863.9
(22) Anmeldetag: 12.09.1997
(51) Int. Cl.: A61K 7/06, A61K 7/11

(54) **Wässrige oder wässrig/alkoholische haarkosmetische Formulierung**

(30) Priorität: 20.09.1996 DE 19638795
(71) Anmelder: BASF AKTIENGESELLSCHAFT, 67056 Ludwigshafen (DE)
(72) Erfinder: Schehlmann, Volker, Dr., 67354 Römerberg (DE); Hössel, Peter, Dr., 67105 Schifferstadt (DE)

(57) **Zusammenfassung**

Wäßrige oder wäßrig/alkoholische haarkosmetische Formulierung enthaltend als Filmbildner Copolymerisate auf (Meth)acrylatbasis mit einem K-Wert von 20 - 150 erhältlich durch radikalische Polymerisation von
(a) 40 - 65 Gew.-% Acrylsäure und/oder Methacrylsäure,
(b) 14 - 60 Gew.-% eines Alkyl(meth)acrylsäureesters oder einer Mischung hieraus und
(c) 0 - 50 Gew.-% eines weiteren radikalisch polymerisierbaren Monomeren oder einer Mischung hieraus,
wobei 20 - 100 % der Carboxylgruppen des Copolymerisats neutralisiert sind.

## Beschreibung

Die vorliegende Erfindung betrifft wäßrige oder wäßrig/alkoholische haarkosmetische Formulierungen enthaltend als Filmbildner Copolymerisate auf (Meth)acrylatbasis.

DE 29 17 504 beschreibt Aerosolhaarspraymassen basierend auf einem Harz, das neben anderen Monomeren zu 5 - 55 Mol% aus carboxylgruppenhaltigen Monomeren besteht. Die Carboxylatgruppen sind neutralisiert mit langkettigen Aminen, wodurch die Löslichkeit des Harzes in Alkohol/Kohlenwasserstofftreibmitteln verbessert wird. Gerade in den ökologisch günstigeren wasserbasierenden Formulierungen sind jedoch langkettige Amine zur Neutralisation nicht geeignet, da sie die Wasserlöslichkeit und Auswaschbarkeit erniedrigen.

DE 43 14 305 beschreibt Haarfestigungsmittel auf Basis von Polymerisaten, die neben tert. Butyl(meth)acrylat noch 10 - 28 Gew.% Acrylsäure oder Methacrylsäure enthalten.

US 4 196 190 beschreibt Haarfestigerharze, die neben drei anderen Monomeren noch 12-30 Gew.% Methacrylsäure enthalten.

EP 590 604 beschreibt eine Acrylatdispersion für die Anwendung in Haarsprays, wobei das Copolymerisat mindestens 60 Gew.-% (Meth)acrylsäureester und 1-15 Gew.-% acrylische Säuren enthält.

Die US 3 577 517 beschreibt Polymere für Haarlacke, die 6 bis 37 Gew.-% Acryl- oder Methacrylsäure enthalten.

Nachteil der oben beschriebenen Copolymerisate ist jedoch, daß zur Erzielung einer hinreichende Auswaschbarkeit vom Haar mehr als 90% der Säuregruppen neutralisiert werden müssen. Dieser hohe Neutralisationsgrad bedingt jedoch, daß der pH der Formulierung Werte größer 8 einnimmt, was insbesondere bei Haarschäumen, Haargelen, Haarlotionen oder Haarfestigerlösung - also bei Formulierungen, die auch intensiv mit der Haut in Berührung kommen, bei empfindlichen Personen zu schwachen Hautreizungen führen kann.

Einen weiterer Nachteil der bekannten acrylsäure- bzw. methacrylsäurehaltigen Copolymerisate ist die vergleichsweise schlechte Trockenkämmbarkeit des behandelten Haares. Zur Verbesserung der Trockenkämmbarkeit werden bisher zusätzliche geeignete Formulierungsbestandteile wie beispielsweise Polysiloxane eingesetzt, die auf der anderen Seite die Kosten der Formulierung erhöhen.

Ziel der vorliegenden Erfindung war es daher, Polymerisate auf Basis von (Meth)acrylsäure zu finden, die in ökologisch verbesserten wasserhaltigen Formulierungen mit einem pH-Wert kleiner 8 verwendet werden können, die sich dennoch gut vom Haar auswaschen lassen und die auch eine so gute Trockenkämmbarkeit besitzen, daß auf den Einsatz von entsprechenden zusätzlichen Hilfsstoffen verzichtet werden kann.

Gefunden wurden wäßrige oder wäßrig/alkoholische haarkosmetische Formulierungen enthaltend als Filmbildner Copolymerisate auf (Meth)acrylatbasis mit einem K-Wert von 20 - 150 erhältlich durch radikalische Polymerisation von
(a) 40 - 65 Gew.-% Acrylsäure und/oder Methacrylsäure,
(b) 14 - 60 Gew.-% eines oder mehrerer Alkyl(meth)acrylsäureesters und
(c) 0 - 50 Gew.-% eines oder mehrerer radikalisch polymerisierbarer Monomeren,
   wobei 20 - 100 % der Carboxylgruppen des Copolymerisats neutralisiert sind.

Besonders bevorzugte haarkosmetische Formulierungen sind solche, bei denen das Copolymerisat aus (a) 45-60 Gew.-% Acrylsäure und/oder Methacrylsäure besteht und bei denen 20- 80 %, insbesondere 20-60 % der Carboxylgruppen des Copolymerisats neutralisiert sind.

Als Alkyl(meth)acrylsäureester (b) kommen in Frage:

Ester der (Meth)acrylsäure mit C₁-C₃₀-Alkylalkoholen, beispielsweise Methylmethacrylat, Ethylacrylat, Ethylmethacrylat, n-Propylacrylat, n-Propylmethacrylat, iso-Propylacrylat, iso-Propylmethacrylat, n-Butylacrylat, n-Butylmethacrylat, iso-Butylacrylat, iso-Butylmethacrylat, t-Butylacrylat, t-Butylmethacrylat, Pentylacrylat, Pentylmethacrylat, n-Hexylacrylat, n-Hexylmethacrylat, n-Heptylacrylat, n-Heptylmethacrylat, n-Octylacrylat, n-Octylmethacrylat, 2-Ethylhexylacrylat, 2-Ethylhexylmethacrylat, Decylacrylat, Decylmethacrylat, Laurylacrylat, Laurylmethacrylat, Palmitylacrylat, Palmitylmethacrylat, Stearylacrylat, Stearylmethacrylat, Hydrenol(meth)acrylat, Behenyl(meth)acrylat, Polyisobuten(meth)acrylat, Phenoxyethylacrylat oder Phenoxyethylmethacrylat, oder um C₅-C₆-Cycloalkyl(meth)acrylate, wobei der Cycloalkylrest durch 1, 2 oder 3 C₁-C₄-Alkylgruppen substituiert sein kann, beispielsweise Cyclohexylacrylat, Cyclohexylmethacrylat, 4-Methylcyclohexylacrylat oder 4-Methylcyclohexylmethacrylat.

Bevorzugt sind C1-C4-Alkyl(meth)acrylate.

Die Ester (b) werden in den Copolymerisaten in einer Menge von 14 - 60, bevorzugt von 20 - 55 Gew.-% bezogen auf das Gesamtgewicht aller Monomere eingesetzt.

Es können als Monomere (b) sowohl Einzelverbindungen als auch Mischungen von Einzelverbindungen eingesetzt werden.

Als radikalisch polymerisierbare Monomere (c) eignen sich vorzugsweise gegebenenfalls N-C1-C18-Alkyl- oder -Hydroxyalkylsubstituierte (Meth)acrylamide, wie Acrylamid, Methacrylamid, N-Methylacrylamid, N-Methylmethacrylamid, N-Ethylacrylamid, N-Ethylmethacrylamid, N-Propylacrylamid, N-Propylmethacrylamid, N-iso-Propylacrylamid, N-iso-Propylmethacrylamid, N-n-Butylacrylamid, N-n-Butylmethacrylamid, N-iso-Butylacrylamid, N-iso-Butylmethacrylamid, N-t-Butylacrylamid, N-t-Butylmethacrylamid, N-Pentylmethacrylamid, N-Hexylacrylamid, N-Hexylmethacrylamid, N-Heptylacrylamid, N-Heptylmethacrylamid, N-Octylacrylamid, N-Octylmethacrylamid, N-2-Ethylhexylacrylamid, N-2-Ethylhexylmethacrylamid, N-Nonylacrylamid, N-Nonylmethacrylamid, N-Decylacrylamid, N-Decylmethacrylamid, N-Laurylacrylamid, N-Laurylmethacrylamid, N-Palmitylacrylamid, N-Palmitylmethacrylamid, Stearylacrylamid, N-Stearylmethacrylamid, N-Hydroxyethylacrylamid, N-Hydroxyethylmethacrylamid, N-Hydroxypropylacrylamid und N-Hydroxypropylmethacrylamid.

Weiterhin eignen sich als Monomere (c):
C₁-C₃₀-Vinylester, wie Vinylacetat, Vinylpropionat, Vinylbutyrat, Vinylvalerat, Vinyl-2-ethylhexanoat, Vinyldecanoat, Vinylpalmitat, Vinylstearat und Vinyllaurat;
C₁-C₃₀-Vinylether, wie Methylvinylether, Ethylvinylether, N-Propylvinylether, iso-Propylvinylether, n-Butylvinylether, iso-Butylvinylether, 2-Ethylhexylvinylether, Decylvinylether, Dodecylvinylether, Stearylvinylether, 2-(Diethylamino)ethylvinylether, 2-(Di-n-Butylamino)ethylvinylether und Methyldiglykolvinylether;
vinylaromatische Verbindungen, z.B. Styrol und substituierte Styrole, wie p-Methylstyrol und a-Methylstyrol, und Si-haltige Monomere, insbesondere ungesättigte Polysiloxane.

Bevorzugte Monomere (c) sind Styrol und Acrylamide.

Die Monomeren (c) werden in den Copolymerisaten in einer Menge von 0 - 50, bevorzugt von 0 - 40 Gew.-%, bezogen auf das Gesamtgewicht aller Monomere eingesetzt.

Es können als Monomere (c) sowohl Einzelverbindungen als auch Mischungen von Einzelverbindungen eingesetzt werden.

Die erfindungsgemäßen Copolymerisate können nach den bekannten Verfahren der Emulsions- Lösungs-, Suspensions- oder Fällungspolymerisation unter Verwendung der dem Fachmann bekannten Hilfsstoffe hergestellt werden.

Die Lösungs- bzw. Emulsionspolymerisate können entweder in einem weiteren Verfahrensschritt in einen Feststoff überführt werden oder direkt in die kosmetische Formulierungen eingesetzt werden.

Der K-Wert (bestimmt in 1 %iger ethanolischer Lösung bei 25°C nach H. Fikentscher, Cellulose-Chemie 13 (1932), S. 58 - 64 und 71, 74) kann durch bekannte Verfahrensvarianten gesteuert werden. Die erfindungsgemäß eingesetzten Copolymerisate haben einen K-Wert von 20 - 150 bevorzugt einen von 30 - 120.

Die Neutralisation der Carboxylgruppen des Copolymerisats kann mit allen gängigen Neutralisierungsmitteln bewirkt werden. Insbesondere sind geeignet Alkalihydroxide, Ammoniak, Alkali- oder Ammoniumcarbonat, C₁ - C₆-mono-, di- oder tri-Alkylamine, Alkanolamine, Tetrahydroxypropylethylendiamin, basische Heterocyclen oder Mischungen dieser Substanzen. Die Neutralisierungsmittel können in reiner oder gelöster Form zugegeben werden. Bevorzugt ist die Zugabe in Form von wäßrigen Lösungen. Besonders bevorzugt sind Alkalihydroxide und 2-Amino-2-methylpropanol-1.

Die Neutralisation der Copolymerisate kann unmittelbar nach der Polymerisation oder erst bei der Formulierung des Haarkosmetikums vorgenommen werden. Die Neutralisation wird in der Regel so vorgenommen, daß das Neutralisierungsmittel zugegeben wird, bis der gewünschte pH-Wert erreicht ist. Im allgemeinen wird ein pH-Wert von 5,5 - 8,0 in der fertigen Formulierung angestrebt.

Die haarkosmetischen Formulierungen enthalten als Lösungsmittel Wasser oder Mischungen aus Wasser und Alkohol, wobei der Wasseranteil in der Mischung üblicherweise 20 Gew.-% oder mehr beträgt.

Zur Modifikation der kosmetischen Eigenschaften können andere filmbildende haarfestigende oder konditionierende Polymere dem Haarkosmetikum zugegeben werden.

Ferner enthalten die haarkosmetischen Formulierungen üblicherweise noch ein oder mehrere der folgenden, dem Fachmann geläufigen Bestandteile:

organische Lösemittel, Treibgase, Gelbildner, Verdicker, Konservierungsmittel, Farbmittel, Parfümöle, Trübungsmittel, Wirkstoffe, UV-Filter und grenzflächenaktive Verbindungen, d.h. Schaumbildner, Emulgatoren, Tenside, Solubilisatoren u.ä. Die eingesetzten grenzflächenaktiven Verbindungen können anionisch, kationisch, amphotär oder neutral sein.

### Beispiele 1-6

### Herstellung von Copolymerisaten

Es wurden 6 verschiedene Copolymerisate hergestellt. Einzelheiten des Herstellungsverfahrens, des Monomerenverhältnisses und der K-Werte sind der Tabelle 1 zu entnehmen.

**Tabelle 1**

| Beispiel | Monomer | Verhältnis (Gew.-%) | Verfahren | K-Wert |
|---|---|---|---|---|
| 1 | EA/MAS | 50/50 | EP | 60 |
| 2 | EA/MAS | 50/50 | EP | 90 |
| 3 | tBA/MAS | 60/40 | EP | 40 |
| 4 | N-tBAM/EA/MAS | 10/30/60 | LP | 35 |
| 5 | tBMA/MAS | 35/65 | FP | 105 |
| 6 | S/AS/MAS/BA | 40/20/25/15 | SP | 66 |

Abkürzung der Monomere:

AS: Acrylsäure; MAS Methacrylsäure; EA: Ethylacrylat; tBA tert.-Butylacrylat; NtBAM: N-tert.-Butylacrylamid; tBMA: tert.-Butylmethacrylat; S: Styrol; BA: Butylacrylat

Abkürzung der Verfahren:

EP: Emulsionspolymerisation: LP: Lösungspolymerisation; SP: Suspensionspolymerisation

### Formulierung von verschiedenen Haarfestigern

Die Copolymerisate der Tabelle 1 wurden in den folgenden Formulierungen eingesetzt und an Modellköpfen, d.h. natürlichen menschlichen Haaren getestet.

Die Prozentangaben sind als Gewichtsprozent zu verstehen.

### (Bsp. 7.1 - 7.6) Formulierung als Haarfestiger

- 35 %: Ethanol
- 3 %: Polymer (aus Beispiel 1 - 6)
- x: Aminomethylpropanol (AMP) pH ca. 7,0
- 0.3 %: PEG40-Hydrogenated Castor Oil (Cremophor RH 40, BASF AG)
- q.s.: Parfümöl
- ad 100 %: Wasser

### (Bsp. 8.1 - 8.6) Formulierung als Fönlotion

- 4 %: Polymer (aus Beispiel 1 - 6)
- x: Aminomethylpropanol (AMP) pH ca. 6,5
- 0.3 %: PEG40-Hydrogenated Castor Oil (Cremophor RH 40, BASF AG)
- q.s.: Parfümöl
- ad 100 %: Wasser

### (Bsp. 9.1 - 9.6) Formulierung als Schaumfestiger

- 5 %: Ethanol
- 3 %: Polymer (aus Beispiel 1 - 6)
- x: Aminomethylpropanol (AMP) pH ca. 6,3
- 0.3 %: Ceteareth 25 (Cremophor A25, BASF AG)
- 10 %: Propan/Butan (25/75)
- ad 100 %: Wasser

Ein Neutralisationsgrad von 60 % (AMP) führt beim besonders bevorzugten Polymer aus Beispiel 1 in einer 3 %igen wäßrigen Lösung zu einem pH-Wert von 6,3.

Bei der anwendungstechnischen Prüfung wurde jeweils ca. 3 g der Formulierung auf eine Halbseite des Modellkopfs aufgetragen.

Beurteilt wurden Haarfestigung, Konditionierwirkung, Trockenkämmbarkeit, Klebrigkeit und Auswaschbarkeit.

Die Formulierungen zeigen eine ausgezeichnete Haarfestigung. Die Trockenkämmbarkeit wird als exzellent beurteilt und das Haar fühlt sich nach Kämmen nicht klebrig an, sondern sehr geschmeidig.

Außerdem lassen sich die Produkte rückstandsfrei durch Waschen mit konventionellen Haarshampoos wieder vom Haar entfernen.

## Patentansprüche

1. Wäßrige oder wäßrig/alkoholische haarkosmetische Formulierung enthaltend als Filmbildner Copolymerisate auf (Meth)acrylatbasis mit einem K-Wert von 20 - 150 erhältlich durch radikalische Polymerisation von
(a) 40 - 65 Gew.-% Acrylsäure und/oder Methacrylsäure,
(b) 14 - 60 Gew.-% eines oder mehrerer Alkyl(meth)acrylsäureester und
(c) 0 - 50 Gew.-% eines oder mehrerer radikalisch polymerisierbarer Monomeren,
wobei 20 - 100 % der Carboxylgruppen des Copolymerisats neutralisiert sind.

2. Haarkosmetische Formulierung nach Anspruch 1, dadurch gekennzeichnet, daß
(a) 45 - 60 Gew.-% Acrylsäure und/oder Methacrylsäure,
(b) 20 - 55 Gew.-% eines oder mehrerer Alkyl(meth)acrylsäureester und
(c) 0 - 40 Gew.-% eines oder mehrerer radikalisch polymerisierbaren Monomeren,
bedeuten und daß 20 - 60 % der Carboxylgruppen des Copolymerisats neutralisiert sind.

3. Haarkosmetische Formulierung nach Anspruch 1oder 2, wobei als Neutralisierungsmittel Alkalihydroxide, Ammoniak, Alkali- oder Ammoniumcarbonat, C1- C6-mono-, di- oder tri-Alkylamine, Alkanolamine, Tetrahydroxypropylethylendiamin, basische Heterocyclen oder Mischungen hieraus eingesetzt werden.

4. Haarkosmetische Formulierung nach einem der voranstehenden Ansprüche, wobei das Copolymerisat durch Emulsions-, Lösungs-, Fällungs- oder Suspensionspolymerisation hergestellt wurde.

5. Haarkosmetische Formulierung nach einem voranstehenden Anspruch, dadurch gekennzeichnet, daß weitere filmbildende haarfestigende und/oder konditionierende Polymerisate zusätzlich enthalten sind.

6. Haarkosmetische Formulierung nach einem voranstehenden Anspruch, dadurch gekennzeichnet, daß es sich um eine Fönlotion, einen Haarfestiger oder einen Schaumfestiger handelt.
